# EUROPEAN PATENT SPECIFICATION

(11) **EP 0 537 955 B1**
(45) Date of publication and mention of the grant of the patent: **11.12.1996**
(21) Application number: 92309230.8
(22) Date of filing: 09.10.1992
(51) Int. Cl.: A61B 17/12, A61B 17/11

(54) **Laparoscopic instrument for the application of endoligatures**
Laparoskopisches Instrument zum Setzen von Endoligaturen
Instrument laparoscopique pour poser des endoligatures

(30) Priority: 12.10.1991 DE 4133800
(43) Date of publication of application: 21.04.1993
(73) Proprietor: ETHICON INC., Somerville New Jersey 08876 (US)
(72) Inventor: Trapp, Rainer, W-7523 Graben-Neudorf (DE); Buess, Gerhard F., Prof. Dr., W-7400 Tubingen/Bebenhausen (DE); Melzer, Andreas, Dr., W-6200 Wiesbaden (DE)
(74) Representative: Mercer, Christopher Paul

(56) References cited:
- EP-A- 0 117 981
- WO-A-90/05491
- WO-A-90/06725
- DE-A- 3 504 202
- DE-U- 9 100 162
- US-A- 3 890 977
- US-A- 5 002 563
- US-A- 5 037 433

## Description

The invention relates to a laparoscopic instrument for the looping of hollow organs and for the application or fixing of endoligatures in the abdominal cavity.

In modern, minimum invasive surgery the instruments necessary for the operation are introduced through one or more trocar cannulas into the abdominal cavity and perform the operation under the control of an endoscope also introduced into said cavity. For the supply of wound areas intraabdominal ligatures are applied, e.g. by endoloops with running knots, which are brought into the area to be ligated with a tubular applicator and are placed round the freely graspable stump of a hollow organ or a vascular bundle. The loop round the stump is tightened from the outside or closed with a ligature clip. The endoscopically controlled application of such an endoligature loop requires considerable manual skill and is also only usable when the vascular stumps can be freely grasped.

In the case of endoscopic operations, considerable difficulties are encountered with conventional instruments in bypassing hollow organs or vascular bundles with a ligature binder, i.e. to form a loop in the abdominal cavity, which can then be tightened from the outside. The difficulties are firstly due to the restricted possibilities of the hitherto used, mainly rod or hook-like instruments and secondly the difficult manual control of such instruments, because the operating field can only be bidimensionally endoscopically seen by the surgeon.

Considerable difficulties have been encountered up to now with the end-to-end anastomosis of hollow organs in laparoscopic surgery. In open surgery it is necessary to apply a purse string suture for performing end-to-end anastomosis with a circular stapling device. Laparoscopically this procedure can only be used under extreme difficulty, because hitherto there has been no procedure available for the automated application of a purse string suture and no corresponding substitute for the latter for laparoscopic applications.

The problem of the invention is to provide a laparoscopic instrument enabling hollow organs to be bypassed under endoscopic conditions and which can be provided with a carried along ligature binder.

According to the invention this problem is solved by a laparoscopic instrument, which is constructed in accordance with the features of claim 1. Advantageous embodiments are given in the subclaims.

An instrument according to the first part of Claim 1 is disclosed in DE-A- 3504202.

The inventive laparoscopic instrument has an instrument tube, which can be introduced through a trocar cannula into the abdominal cavity. A spiral spring element and a gripping tool are located in the instrument tube and both can be moved independently of one another in the longitudinal direction of said tube with the aid of rod-slide mechanisms and can be slid out of the distal end of the instrument tube into the abdominal cavity. The spiral spring element is under tension in the unrolled state in said instrument tube. The length and resiliency of the spiral spring element are so dimensioned that in the tension-free state it forms an approximately circular turn or winding, so that the slid out end of the spring element in the abdominal cavity returns just upstream of the instrument tube end. Thus, on sliding out, the spiral spring element bypasses a hollow organ upstream of the distal end of the instrument tube. A suitable ligature binder in the instrument tube and whose front end is fixed to the front of the spiral spring element is passively carried along by the spiral spring element and consequently loops the hollow organ in one turn. The displaceable gripping tool grips the ligature binder end upstream of the instrument tube under the guidance of the surgeon and draws it back into the said tube in order to thread it outside and move the closure to the ligature point, or retains it for use elsewhere.

In a preferred embodiment the spiral spring element is made from so-called superelastic material. These materials, which are also called shape memory metals, have high resiliency characteristics and, compared with other spring materials, can be much more highly loaded without undergoing plastic deformation and losing the impressed winding shape. Thus, a strip-like spiral spring element made from such a material can be kept rolled out in the instrument tube without losing the impressed winding shape, which it adopts with high resiliency after sliding out. Nickel-titanium alloys form a preferred group of superelastic materials.

In a preferred embodiment the spiral spring element is kept unrolled by a tubular hollow body displaceably located within the instrument tube. The hollow body is slid out of the distal end of the instrument tube and then the spiral spring element is moved out of the distal end of the hollow body by means of the slide mechanism, in order to return to the relaxed form and form a winding.

The gripping tool which can be slid out is constituted by gripping forceps in a preferred embodiment. This embodiment is particularly suitable for the use of plastic strips as the ligature binders. A suitable plastic strip is provided at one end with a ring and has over its longitudinal extension notches or is laterally given a toothed structure, which stop the binder on drawing the other end through the ring. In this embodiment the plastic strip is placed longitudinally in the instrument tube and is fixed by the ring or tip to the front end of the spiral spring element, to whose tip is fitted a retaining device. The retaining device can be formed by a hook-like bending of the tip, which retains the binder ring, or by a clamp fitted to the tip and which clamps the binder tip.

On sliding out the spiral spring the binder is carried along and looped round the hollow vessel. The binder end can then be gripped with the gripping forceps and drawn out through the instrument tube. The binder ends can be gripped outside the instrument tube and one can be drawn through the ring. The ring is then moved through the instrument tube back to the structure to be ligated, optionally using sliding forceps, and in this way the loop is secured round the hollow organ.

In an alternative embodiment the gripping tool is constituted by a hook at the end of the slide mechanism. This embodiment is particularly suitable when using ligatures. The ligature is looped round the hollow organ with the spiral spring element, on whose tip it is held in a suitable eye, and is subsequently gripped by the hook and drawn to the instrument tube. For closing the ligature it is either possible to fit, in known manner, a ligature clip, or the ligature end can be retracted entirely through the instrument tube, to be externally provided with a running knot, which can subsequently be tightened for closing the ligature.

The described instrument is also suitable for use in open surgery when performing an end-to-end anastomosis with stapling devices and can there replace the purse string suture.

The invention is described in greater detail hereinafter relative to an embodiment and the attached drawings, wherein show:

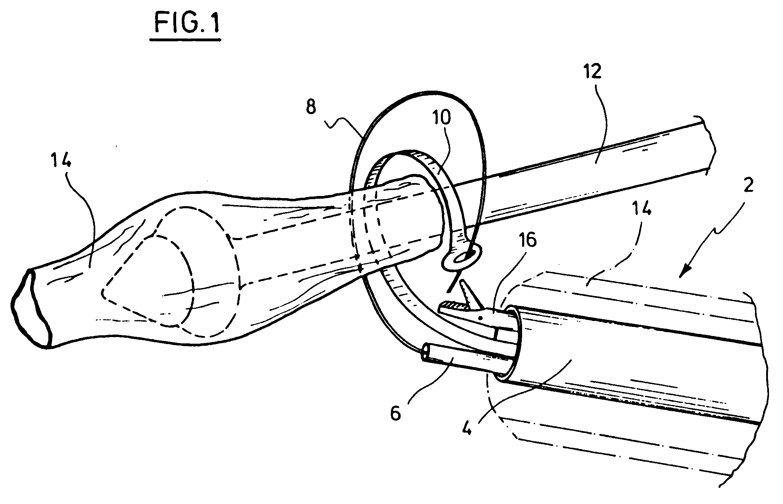

Fig. 1 a perspective view of the distal end of an instrument according to the invention when applying an endoligature in an intestinal operation.
Fig. 2 an end-to-end anastomosis after tightening the plastic ligature strips around the intestine and a central pin.
Figs. 3 and 4 a plastic ligature strip with a sawtooth structuring of the lateral faces for stopping in the ring.
Fig. 5 a plastic ligature strip with notch structuring of one surface for stopping in the ring.

The embodiment shown in the drawings is particularly appropriate for the use of plastic ligature strips. The instrument tube 4 of the instrument according to the invention is advanced through a trocar cannula 14 by its distal end to the operating field. It is possible to see part of the intestine 14, in which is inserted a central pin 12 and which is to be fixed in this operating stage to the pin, in that a plastic strip is secured over the intestine and the central pin. The instrument tube 4 is provided with a longitudinally displaceable slide mechanism 6, to whose tip is fitted the spiral spring element 8. In the drawn-in state the spiral spring element is held rolled out under tension in the longitudinal direction of the instrument tube, e.g. in a not shown tubular hollow body surrounding the spiral spring strip. If the instrument tube is moved close up to the structure to be looped, the spiral spring element is slid out by means of the slide mechanism 6 and then returns to the preimpressed, relaxed form with approximately one winding. To the tip of the spiral spring element a plastic strip 10 is attached to a not shown retaining device and is passively passed round the intestine with the spiral spring element 8. The front end of the plastic strip 10 is provided with a ring and its rear end extends out of the instrument tube 4.

When the spiral spring element has guided the plastic strip 10 about the structure to be bypassed and back close to the distal end of the instrument tube, the end of the plastic strip can be gripped with a gripping forceps 16. The latter is operable from the outside by means of a known mechanism and can be slid in the longitudinal direction of the instrument tube on a slide mechanism. When the gripping forceps 16 has gripped the front end of the plastic strip 10, then the surgeon draws the front plastic strip end through the instrument tube to the outside, where the rear plastic strip end can easily be drawn through the ring. The plastic binder can then be tightened, optionally using an appropriate instrument, per se known sliding forceps with an adapted diameter and length being particularly suitable for this purpose. The sliding forceps grips the plastic strip, slides the head forward during compression and thus stepwise closes the plastic strip loop until, with the binder contracted, the forceps meets a greater resistance, when the projecting plastic binder end is separated.

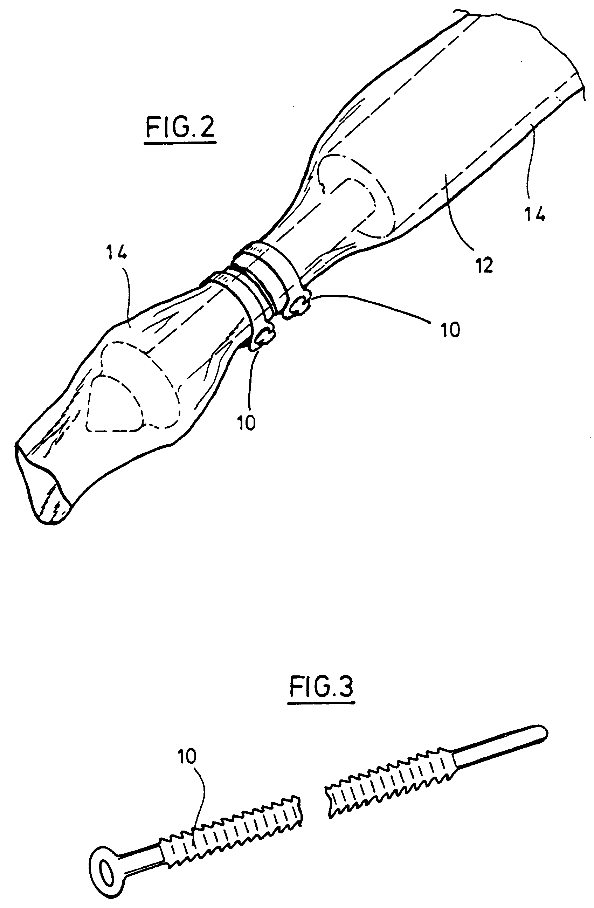

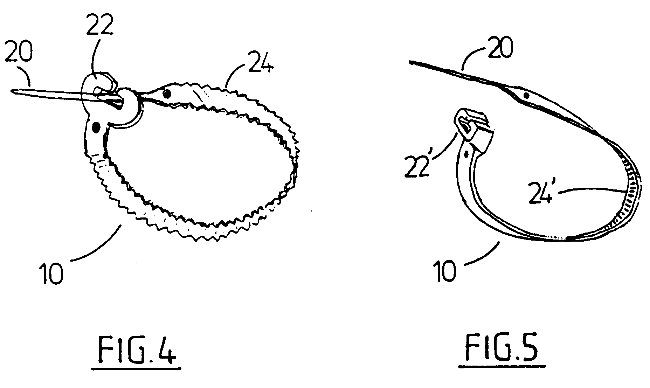

The construction of a suitable plastic binder is shown in fig. 3. The binder is constructed as a plastic strip, which is laterally given a sawtooth structure. At the front end a ring is provided through which the facing binder end is drawn in order to close the binder. The lateral sawtooth structuring is such that on contracting the binder it slides through as a result of the flexibility of the plastic material, but is positively stopped on sliding back due to its shape and in this way reliably closes the binder. Fig. 4 shows a binder 10 particularly appropriate for applying an endoligature loop and which is laterally provided with a notch-like sawtooth structuring 24. At one end the binder has an elongated, thin tip 20 and at the other end a ring 22, which is not completely closed, instead being provided with a slit through which the elongated tip 20 can be introduced laterally into the ring opening. This renders it superfluous to thread and draw one binder end through the ring, which means a significant simplification under operating conditions.

A tooth or notch structuring 24′ can also be provided on one flat side of the binder 10, as shown in fig. 5. In this case the ring as a notch tip 22′ is provided with a stop member, which engages in the notch gaps and consequently prevents sliding back and fixes the binder. Once again an elongated tip 20 is provided, which can be inserted through a slit in the notch tip, so that once again there is no need for a complicated threading operation.

A typical use of the inventive instrument is illustrated in fig. 2 on removing part of the intestine. Here a central pin 12 is inserted in the intestine 14 and the facing intestine ends are secured onto the pin with the aid of plastic strips 10. In this case the plastic strips 10 replace the purse string suture known from open surgery and consequently such operations can also be carried out using minimum invasive surgery.

## Claims

1. Laparoscopic instrument for looping hollow organs and for the application of endoligatures, particularly in the abdominal cavity, comprising an instrument tube (4), insertable through a trocar cannula (14) with a distal end into the abdominal cavity, and a gripping tool (16) longitudinally movable in the instrument tube and which can be slid out of the distal end and which serves to grip a ligature binder characterised in that it comprises a spiral spring element (8) which, under loading, is guided in the unrolled state in the longitudinal direction of the instrument tube and can be slid by means of a slide mechanism (6) out of the distal end, the spiral spring element being dimensioned in such a way that its resiliency returns the slid-out end with one winding to the distal end of the instrument tube and in that the gripping tool (16) serves to grip the ligature binder carried along by the spiral spring element.

2. Laparoscopic instrument according to claim 1, characterized in that the spiral spring element (8) is produced from a superelastic material strip, which forms a winding in the unloaded state.

3. Laparoscopic instrument according to claim 2, characterized in that the spiral spring element (8) is produced from a nickel-titanium alloy.

4. Laparoscopic instrument according to one of the preceding claims, characterized in that the spiral spring element (8) in the instrument tube is held unrolled in a tubular hollow body, which is displaceably guided in the instrument tube, it being possible to slide the spiral spring element out of the hollow body through the slide mechanism (6).

5. Laparoscopic instrument according to one of the preceding claims, characterized in that the gripping tool is constructed as gripping forceps (16).

6. Laparascopic instrument according to one of the preceding claims, characterized in that at its distal end the spiral spring element has a device for holding the ligature binder.

7. Laparoscopic instrument according to claim 6, characterized in that the device for holding the ligature binder is formed by a hook-like bend.

8. Laparoscopic instrument according to claim 6, characterized in that the device for holding the ligature binder is formed by a laterally bent clamping device.

9. Laparoscopic instrument according to one of the preceding claims, characterized in that the gripping tool is constructed as a hook for gripping a ligature binder.

10. Laparoscopic instrument according to one of the preceding claims, characterized in that a clip applicator, which can be slid out, can be introduced into the instrument tube (4).

11. Laparoscopic instrument according to one of the preceding claims, characterized in that a longitudinally displaceable closing forceps can also be introduced into the instrument tube (4).

## Patentansprüche

1. Laparoskopisches Instrument zum Umschlingen von hohen Organen und zum Setzen von Endoligaturen, insbesondere in der Bauchhöhle, mit einem Instrumentenrohr (4), das durch eine Trokarkanüle (14) mit dem distalen Ende in die Bauchhöhle einsetzbar ist, und einem Greifwerkzeug (16), das in dem Instrumentenrohr in der Längsrichtung verschiebbar ist und aus dem distalen Ende herausgeschoben werden kann, und das dazu dient, einen Ligaturbinder zu ergreifen, dadurch gekennzeichnet, daß es ein Spiralfederelement (8) aufweist, das unter Belastung in dem entrollten Zustand in der Längsrichtung des Instrumentenrohrs geführt wird und mittels eines Gleitmechanismus (6) aus dem distalen Ende herausgeschoben werden kann, wobei das Spiralfederelement so dimensioniert ist, daß sein herausgeschobenes Ende infolge der Elastizität des Spiralfederelements nach einer Windung zu dem distalen Ende des Instrumentenrohrs zurückkehrt, und daß das Greifwerkzeug (16) dazu dient, den Ligaturbinder zu ergreifen, der längs des Spiralfederelements mitgeführt wird.

2. Laparoskopisches Instrument gemäß Anspruch 1, dadurch gekennzeichnet, daß das Spiralfederelement (8) aus einem superelastischen Materialstreifen hergestellt ist, der in dem unbelasteten Zustand eine Windung bildet.

3. Laparoskopisches Instrument gemäß Anspruch 2, dadurch gekennzeichnet, daß das Spiralfederelement (8) aus einer Nickel-Titan-Legierung hergestellt ist.

4. Laparoskopisches Instrument gemäß irgendeinem der vorhergehenden Ansprüche, dadurch gekennzeichnet, daß das Spiralfederelement (8) in dem Instrumentenrohr in einem rohrförmigen, hohen Körper, der in dem Instrumentenrohr verschiebbar geführt wird, im entrollten Zustand gehalten wird, wobei es möglich ist, das Spiralfederelement durch den Gleitmechanismus (6) hindurch aus dem hohlen Körper herauszuschieben.

5. Laparoskopisches Instrument gemäß irgendeinem der vorhergehenden Ansprüche, dadurch gekennzeichnet, daß das Greifwerkzeug als Greifzange (16) verwirklicht ist.

6. Laparoskopisches Instrument gemäß irgendeinem der vorhergehenden Ansprüche, dadurch gekennzeichnet, daß das Spiralfederelement an seinem distalen Ende eine Vorrichtung zum Festhalten des Ligaturbinders hat.

7. Laparoskopisches Instrument gemäß Anspruch 6, dadurch gekennzeichnet, daß die Vorrichtung zum Festhalten des Ligaturbinders von einem hakenähnlichen Ende gebildet wird.

8. Laparoskopisches Instrument gemäß Anspruch 6, dadurch gekennzeichnet, daß die Vorrichtung zum Festhalten des Ligaturbinders von einer seitlich gebogenen Klemmvorrichtung gebildet wird.

9. Laparoskopisches Instrument gemäß irgendeinem der vorhergehenden Ansprüche, dadurch gekennzeichnet, daß das Greifwerkzeug als Haken zum Ergreifen eines Ligaturbinders verwirklicht ist.

10. Laparoskopisches Instrument gemäß irgendeinem der vorhergehenden Ansprüche, dadurch gekennzeichnet, daß ein
Clip-Applikator, der herausgeschoben werden kann, in das Instrumentenrohr (4) eingeführt werden kann.

11. Laparoskopisches Instrument gemäß irgendeinem der vorhergehenden Ansprüche, dadurch gekennzeichnet, daß eine in der Längsrichtung verschiebbare Schließzange ebenfalls in das Instrumentenrohr (4) eingeführt werden kann.

## Revendications

1. Instrument laparoscopique pour cercler des organes creux et pour l'application d'endoligatures, en particulier dans la cavité abdominale, comportant un tube d'instrument (4), pouvant être inséré à travers une canule de trocart (14) ayant une extrémité distale située à l'intérieur de la cavité abdominale, et un outil de saisie (16) mobile longitudinalement dans le tube d'instrument et qui peut coulisser à l'extérieur de l'extrémité distale et qui sert à saisir un lien de ligature, caractérisé en ce qu'il comporte un élément formant ressort en spirale (8) qui, lorsqu'il est sous charge, est guidé dans l'état déroulé dans la direction longitudinale du tube d'instrument et peut coulisser à l'aide d'un mécanisme de coulissement (6) à l'extérieur de l'extrémité distale, l'élément formant ressort en spirale ayant des dimensions telles qu'il ramène élastiquement l'extrémité ayant coulissé à l'extérieur sous forme d'un enroulement vers l'extrémité distale du tube d'instrument et en ce que l'outil de saisie (16) sert à saisir le lien de ligature entraîné par l'élément formant ressort en spirale.

2. Instrument laparoscopique selon la revendication 1, caractérisé en ce que l'élément formant ressort en spirale (8) est constitué d'une bande de matériau super-élastique, qui forme un enroulement à l'état déchargé.

3. Instrument laparoscopique selon la revendication 2, caractérisé en ce que l'élément formant ressort en spirale (8) est constitué d'un alliage nickel-titane.

4. Instrument laparoscopique selon l'une quelconque des revendications précédentes, caractérisé en ce que l'élément formant ressort en spirale (8) situé dans le tube d'instrument est maintenu déroulé dans un corps tubulaire creux, qui est guidé de manière à pouvoir être déplacé dans le tube d'instrument, l'élément formant ressort en spirale pouvant être amené à coulisser à l'extérieur du corps creux par l'intermédiaire du mécanisme de coulissement (6).

5. Instrument laparoscopique selon l'une quelconque des revendications précédentes, caractérisé en ce que l'outil de saisie est construit en tant que forceps de saisie (16).

6. Instrument laparoscopique selon l'une quelconque des revendications précédentes, caractérisé en ce qu'au niveau de son extrémité distale l'élément formant ressort en spirale a un dispositif pour maintenir le lien de ligature.

7. Instrument laparoscopique selon la revendication 6, caractérisé en ce que le dispositif pour maintenir le lien de ligature est formé par une extrémité en forme de crochet.

8. Instrument laparoscopique selon la revendication 6, caractérisé en ce que le dispositif pour maintenir le lien de ligature est formé par un dispositif de serrage courbé latéralement.

9. Instrument laparoscopique selon l'une quelconque des revendications précédentes, caractérisé en ce que l'outil de saisie est constitué sous forme d'un crochet pour saisir un lien de ligature.

10. Instrument laparoscopique selon l'une quelconque des revendications précédentes, caractérisé en ce qu'un applicateur de clip, qui peut coulisser, peut être introduit à l'intérieur du tube d'instrument (4).

11. Instrument laparoscopique selon l'une quelconque des revendications précédentes, caractérisé en ce des forceps de fermeture pouvant être déplacés longitudinalement peuvent aussi être introduits à l'intérieur du tube d'instrument (4).
